(19) Europäisches Patentamt
European Patent Office
Office européen des brevets

(11) EP 0 946 513 B1

(12) **EUROPÄISCHE PATENTSCHRIFT**

(45) Veröffentlichungstag und Bekanntmachung des Hinweises auf die Patenterteilung:
**30.01.2002 Patentblatt 2002/05**

(21) Anmeldenummer: **97953830.3**

(22) Anmeldetag: **16.12.1997**

(51) Int Cl.[7]: **C07D 221/12**, C09K 19/34, C09K 19/40, C07F 7/08

(86) Internationale Anmeldenummer:
**PCT/EP97/07058**

(87) Internationale Veröffentlichungsnummer:
**WO 98/27060 (25.06.1998 Gazette 1998/25)**

(54) **6-FLUORPHENANTHRIDIN-DERIVATE FÜR FLÜSSIGKRISTALLINE MISCHUNGEN**
DERIVATIVES OF 6-FLUOROPHENANTHRIDINE FOR LIQUID CRYSTAL MIXTURES
DERIVES DE 6-FLUOROPHENANTHRIDINE POUR MELANGES DE CRISTAUX LIQUIDES

(84) Benannte Vertragsstaaten:
**DE**

(30) Priorität: **19.12.1996 DE 19653009**

(43) Veröffentlichungstag der Anmeldung:
**06.10.1999 Patentblatt 1999/40**

(73) Patentinhaber: **Aventis Research & Technologies GmbH & Co. KG**
**65926 Frankfurt am Main (DE)**

(72) Erfinder:
- **SCHMIDT, Wolfgang**
**D-51143 Köln (DE)**
- **MANERO, Javier**
**D-65835 Liederbach (DE)**

(74) Vertreter: **Isenbruck, Günter, Dr. et al**
**Patent- und Rechtsanwälte,**
**Bardehle-Pagenberg-Dost-Altenburg-Geissler-Isenbruck Theodor-Heuss-Anlage 12**
**68165 Mannheim (DE)**

(56) Entgegenhaltungen:
**WO-A-95/21227**
**DE-A- 19 500 768**
**DE-A- 19 517 060**

EP 0 946 513 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Neben nematischen und cholesterischen Flüssigkristallen werden in jüngerer Zeit auch optisch aktive geneigt smektische (ferroelektrische) Flüssigkristalle in kommerziellen Displayvorrichtungen verwendet.

Clark und Lagerwall konnten zeigen, daß der Einsatz ferroelektrischer Flüssigkristalle (FLC) in sehr dünnen Zellen zu optoelektrischen Schalt-oder Anzeigeelementen führt, die im Vergleich zu den herkömmlichen TN ("twisted nematic")-Zellen um bis zu einem Faktor 1000 schnellere Schaltzeiten haben (siehe z.B. EP-A 0 032 362). Aufgrund dieser und anderer günstiger Eigenschaften, z.B. der bistabilen Schaltmöglichkeit und des nahezu blickwinkelunabhängigen Kontrasts, sind FLCs grundsätzlich für Anwendungsgebiete wie Computerdisplays gut geeignet.

Für die Verwendung von FLCs in elektrooptischen oder vollständig optischen Bauelementen benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Zur Erzielung eines guten Kontrastverhältnisses in elektrooptischen Bauelementen ist eine einheitliche planare Orientierung der Flüssigkristalle nötig. Eine gute Orientierung in der $S_A$ und $S^*_C$-Phase läßt sich z.B. erreichen, wenn die Phasenfolge der Flüssigkristallmischung mit abnehmender Temperatur lautet:

$$\text{Isotrop - } N^* \rightarrow S_A \rightarrow S^*_C$$

Voraussetzung ist, daß der Pitch (Ganghöhe der Helix) in der N*-Phase sehr groß (größer 10 µm) oder, noch besser, völlig kompensiert ist (siehe z.B. T. Matsumoto et al., Proc. of the 6th Int. Display Research Conf., Japan Display, Sept. 30- Okt. 2, 1986, Tokyo, Japan, S. 468-470; M. Murakami et al., ibid. S. 344 -S. 347). Dies erreicht man z.B., indem man zu der chiralen Flüssigkristallmischung, die in der N*-Phase z.B. eine linksdrehende Helix aufweist, einen oder mehrere optisch aktive Dotierstoffe, die eine rechtsdrehende Helix induzieren, in solchen Mengen hinzugibt, daß die Helix kompensiert wird.

Für die Verwendung des SSFLCD-Effektes (Surface Stabilized Ferroelectric Liquid Crystal Display) von Clark und Lagerwall zur einheitlichen, planaren Orientierung ist femer Voraussetzung, daß der Pitch in der smektischen $C^*$-Phase wesentlich größer ist als die Dicke des Anzeigeeiementes (Mol. Cryst. Liq. Cryst. 1983, 94, 213 und 1984, 1.14, 151).

Die optische Schaltzeit T [µs] ferroelektrischer Flüssigkristallsysteme, die möglichst kurz sein soll, hängt von der Rotationsviskosität des Systems γ [mPas], der spontanen Polarisation $P_s$[nC/cm$^2$] und der elektrischen Feldstärke E[V/m] ab nach der Beziehung

$$T\text{-}\ \frac{Y}{P_s \cdot E}$$

Da die Feldstärke E durch den Elektrodenabstand im elektrooptischen Bauteil und durch die angelegte Spannung festgelegt ist, muß das ferroelektrische Anzeigemedium niedrigviskos sein und eine hohe spontane Polarisation aufweisen, damit eine kurze Schaltzeit erreicht wird.

Schließlich wird neben thermischer, chemischer und photochemischer Stabilität eine kleine optische Anisotropie Δn und eine geringe positive oder vorzugsweise negative dielektrische Anisotropie Δε verlangt (siehe z.B. S.T. Lagerwall et al., "Ferroelectric Liquid Crystals for Displays" SID Symposium, Oct. Meeting 1985, San Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge $I \rightarrow N \rightarrow S_A \rightarrow S_C$ aufweisen. Weitere Komponenten der Mischung werden oftmals zur Schmelzpunktserniedrigung und zur Verbreiterung der $S_C$-und meist auch N-Phase, zum Induzieren der optischen Aktivität, zur Pitch-Kompensation und zur Anpassung der optischen und dielektrischen Anisotropie zugesetzt, wobei aber beispielsweise die Rotationsviskosität möglichst nicht vergrößert werden soll.

Ferroelektrische Flüssigkristallanzeigen lassen sich auch durch Nutzung des DHF (Distorted Helix Formation)-Effektes oder des PSFLCD-Effektes (Pitch Stabilized Ferroelectric Liquid Crystal Display, auch SBF = Short Pitch Bistable Ferroelectric Effect genannt) betreiben. Der DHF-Effekt wurde von B.I. Ostrovski in Advances in Liquid Crystal Research and Applications, Oxford/Budapest 1980, 469ff. beschrieben, der PSFLCD-Effekt ist in DE-A 39 20 625 bzw. EP-A 0 405 346 beschrieben. Zur Nutzung dieser Effekte wird im Gegensatz zum SSFLCD-Effekt ein flüssigkristallines Material mit einem kurzen $S_C$-Pitch benötigt.

In den Positionen 4 und/oder 7 fluorierte Phenanthridin-Derivate sind als Flüssigkristalle bzw. als Komponenten flüssigkristalliner Mischungen aus DE-A-44 02 986 bekannt.

Derivate des Phenanthrens und des 9,10-Dihydrophenanthrens wurden bereits als Flüssigkristalle bzw. als Komponenten flüssigkristalliner Mischungen beschrieben: Azomethine mit einer Phenanthren- bzw. 9,10-Dihydrophenanthren-Einheit sind aus *J. Chem.* Soc. **1958,** 552 und *J. Chem. Soc., Perkin Trans. II* **1982,** 465-472 bekannt; Keto-Derivate des 9,10-Dihydrophenanthrens sind aus *Chem. Ind*. [London] **1974,** 615, *Prod. Int. Liq. Cryst. Conf.* **1973,** 397 und *Tetrahedron* **1981,** 37, 2815 bekannt; Carboxyl-Derivate des 9,10-Dihydrophenanthrens sind aus DD-WP 153 826 und *Z. Chem.* **1983,** *23,* 21-22 bekannt; 2,7-Bis(alkoxy)phenanthrene sind aus *Nippon Kagaku Kaishi* **1980,** 250 bekannt; Phenanthrenderivate mit einem oder mehreren Fluorsubstituenten in den Position 1, 3, 4, 5, 6 und 8 sind aus DE-A 19 500 768 bekannt.

Da aber die Entwicklung, insbesondere von ferroelektrischen Flüssigkristallmischungen, noch in keiner Weise als abgeschlossen betrachtet werden kann, sind die Hersteller von Displays an den unterschiedlichsten Komponenten für Mischungen interessiert. Dieses u. a. auch deshalb, weil erst das Zusammenwirken der flüssigkristallinen Mischungen mit den einzelnen Bauteilen der Anzeigevorrichtung bzw. der Zellen (z. B. der Orientierungsschicht) Rückschlüsse auf die Qualität auch der flüssigkristallinen Mischungen zuläßt.

Aufgabe der vorliegenden Erfindung war es daher, Verbindungen bereitzustellen, die in flüssigkristallinen Mischungen geeignet sind, das Eigenschaftsprofil dieser Mischungen zu verbessern.

Es wurde nun überraschend gefunden, daß 6-Fluorphenanthridin-Derivate der Formel (I) in besonderer Weise zum Einsatz in Flüssigkristallmischungen geeignet sind.

Ein Gegenstand der Erfindung sind daher 6-Fluorphenanthridin-Derivateder Formel (I),

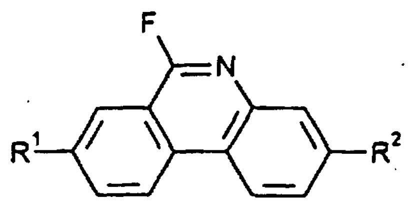

(I)

in der die Symbole folgende Bedeutungen haben:

$R^1$, $R^2$ sind gleich oder verschieden ein geradkettiger oder verzweigter Alkylrest (mit oder ohne asymmetrisches C-Atom) mit 1 bis 16 C-Atomen, wobei

- b1) eine oder zwei nicht benachbarte und nicht terminale $CH_2$-Gruppen durch -O-, -CO-O-, -O-CO-, -O-CO-O- oder $-Si(CH_3)_2$-ersetzt sein können und/oder
- b2) eine $CH_2$-Gruppe durch 1,4-Phenylen oder trans-1,4-Cyclohexylen ersetzt sein kann und/oder
- b3) ein oder mehrere H-Atome durch -F ersetzt sein können und/oder
- b4) die terminale $CH_3$-Gruppe durch eine der folgenden chiralen Gruppen (optisch aktiv oder racemisch) ersetzt sein kann:

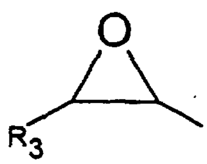

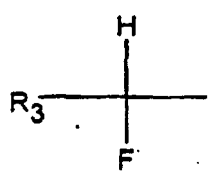

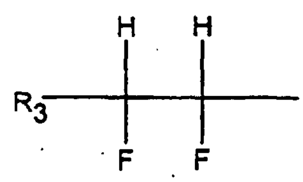

$R^3$ ist

- a) Wasserstoff,
- b) ein geradkettiger oder verzweigter Alkylrest (mit oder ohne asymmetrisches C-Atom) mit 1-14 C-Atomen, wobei
  - b1) eine nicht terminale $CH_2$-Gruppe durch -O- ersetzt sein kann.

Mit der Bereitstellung von Verbindungen der Formel (I) wird ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

In diesem Zusammenhang besitzen die Verbindungen der Formel (I) einen breiten Anwendungsbereich. In Abhängigkeit von der Auswahl der Substituenten können sie als Basismaterialien dienen, aus denen flüssigkristalline Phasen zum überwiegenden Teil zusammengesetzt sind; es können aber auch Verbindungen der Formel (I) flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Besonders geeignet sind die Verbindungen der Formel (I), um schon in geringen Zumischmengen die dielektrische Anisotropie ($\Delta\varepsilon$) in Richtung auf höhere negative Werte zu beeinflussen.

Die erfindungsgemäßen Verbindungen der Formel (1) eignen sich insbesondere zur Verwendung in FLC-Mischungen für ferroelektrische Schalt- und/oder Anzeigevorrichtungen, die im Inverse-Mode betrieben werden.

Die Herstellung der erfindungsgemäßen Verbindungen erfolgt nach an sich literaturbekannten Methoden, wie sie in Standardwerken zur Organischen Synthese, z. B. Houben-Weyl, Methoden der Organischen Chemie, Georg-Thieme-Veriag, Stuttgart, beschrieben werden.

Die Herstellung erfolgt dabei unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) umsetzt.

Beispielhaft sind in den Schemata 1 und 2 Synthesewege zu 6-Fluorphenanthridinen und Verbindungen der Formel (I) angegeben, wobei auch andere Verfahren denkbar und möglich sind.

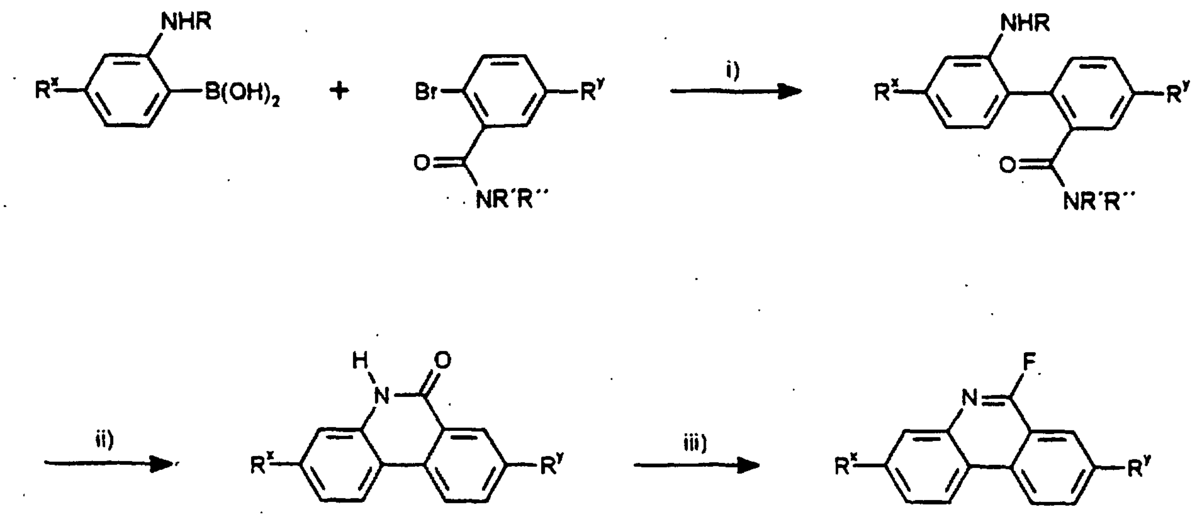


**Schema 1**

Synthese von Verbindungen der Formel (I) über Boronsäurekupplung. R = *t*-BOC; R' = Alkyl, R" = H, Alkyl. i) $Pd^0$-Kat., *Tetrahedron Lett.* **1988,** 29, 5463-5466, EP-A 694 530; ii) $CF_3COOH$, *Tetrahedron Lett.* **1988,** *29,* 5463-5466; iii) DAST/$CH_2Cl_2$, analog EP-A 0 475 444.

Eine alternativer Syntheseweg (Schema 2) führt ausgehend von Fluorenonen zu Verbindungen der Formel (I).

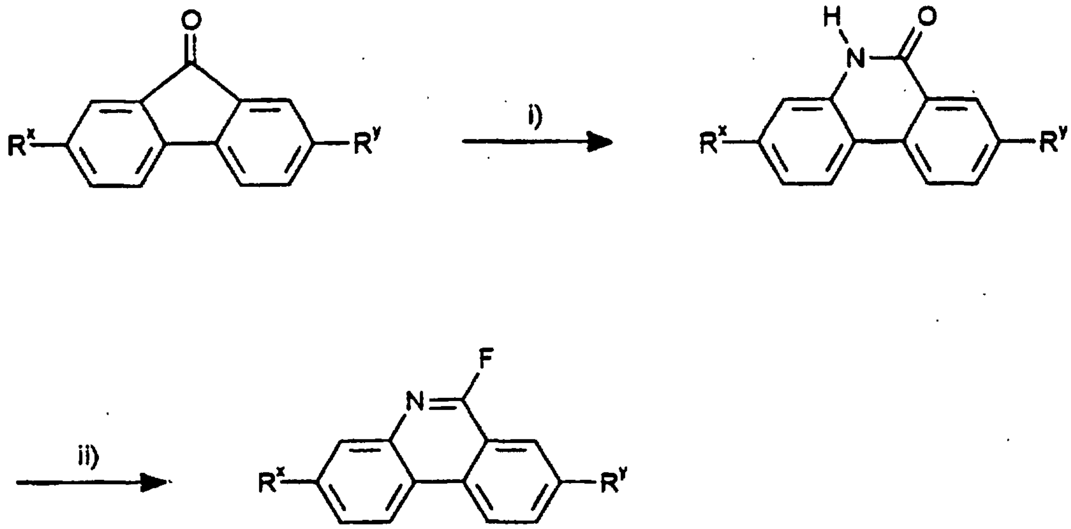


Schema 2

Synthese von Verbindungen der Formel (I) durch Schmidt-Umlagerung entsprechender Fluorenone. i) $NaN_3/H_2SO_4/CHCl_3$, *J. Chem. Soc.* **1965,** 3032-3037 oder $NaN_3/H_2SO_4/CCl_3COOH$, *Tetrahedron* **1971,** 27, 3405-3416; ii) $DAST/CH_2Cl_2$, analog EP-A 0 475 444.

Auch die Umsetzung der entsprechenden Oxime mit Polyphosphorsäure (Beckmann-Umlagerung) führt zu 6-Phenanthridonen (*J. Chem. Soc.* **1965,** 3032-3037).

Die Synthese der entsprechenden Fluorenone kann beispielsweise ausgehend von käuflichem 2,7-Dibromfluorenon oder nach *Tetrahedron* **1971,** *27*, 3405-3416 erfolgen.

Die bei der Umlagerung von Fluorenonen mit unsymmetrischer Substitution ($R^x \neq R^y$) möglicherweise entstehenden Stellungsisomere (Schema 2) lassen sich anschließend oder auf einer der nachfolgenden Stufen z. B. säulenchromatographisch trennen.

Die Gruppe $R^x$ ist gleich der Gruppierung $R^2$ oder eine geeignete, gegebenenfalls geschützte Vorstufe hiervon, die in späteren Schritten nach an sich bekannten, dem Fachmann geläufigen Methoden in diese Gruppierung überführt werden kann:

Die Gruppe $R^y$ ist gleich der Gruppierung $R^1$ oder eine geeignete, gegebenenfalls geschützte Vorstufe hiervon, die in späteren Schritten nach an sich bekannten, dem Fachmann geläufigen Methoden in diese Gruppierung überführt werden kann.

Die Synthese des Restes $R^x$ bzw. $R^y$ erfolgt nach an sich bekannten, dem Fachmann geläufigen Methoden. Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch machen.

Beispielsweise sei verwiesen auf DE-A 23 44 732, 24 50 088, 24 29 093, 25 02 94, 26 36 684, 27 01 591 und 27 52 975 für Verbindungen mit 1,4-Cyclohexylen oder 1,4-Phenylen-Gruppen; DE-A 26 41 724 für Verbindungen mit Pyrimidin-2,5-diyl-Gruppen; DE-A 40 26 223, EP-A 0 391 203 und die deutsche Patentanmeldung 196 48 171.6 für Verbindungen mit Pyridin-2,5-diyl-Gruppen; DE-A 32 31 462 für Verbindungen mit Pyridazin-3,6-diyl-Gruppen; EP-A 309 514 für Verbindungen mit (1,3,4)-Thiadiazol-2-5-diyl-Gruppen und EP-A 0 630 903 für Verbindungen mit 1-Sila-1,4-cyclohexylen-Gruppen.

Ferner sei verwiesen auf *Tetrahedron Lett.* **1981,** 37, 2815-1821, *ibid.* **1985,** 26, 61-64 und *Z. Chem.* **1983,** *23,* 21-22 für die Einführung von Alkyl- und Acylsubstituenten, *Bioorg*: *Med. Chem. Lett*, **1996,** *6*, 481-484 für die Einführung von Brom-, Chlor- und Carboxysubstituenten in den Positionen 2 und 7 eines 9,10-Dihydrophenanthrengerüsts.

2,3-Difluorphenylboronsäure und 4-Alkoxy-2,3-difluorphenylboronsäuren sind beispielsweise in WO 96/00710 beschrieben; auf analoge Weise kann auch 4-Benzyloxy-2,3-difluorphenylboronsäure erhalten werden (WO 96/01246). Beispiele für 4-alkylsubstituierte 2,3-Difluorphenylboronsäuren finden sich in EP-A 0 363 458.

Verbindungen, worin ein aromatischer Ring durch mindestens ein F-Atom substituiert ist, können auch aus den entsprechenden Diazoniumsalzen durch Austausch der Diazoniumgruppe gegen ein Fluoratom, z. B. nach den

Methoden von Balz und Schiemann, erhalten werden.

Was die Verknüpfung der Ringsysteme miteinander angeht, sei beispielsweise verwiesen auf: N. Miyaura, T. Yanagai und A. Suzuki in *Synthetic Communications* **1981,** *11*, 513-519; DE-C 39 30 663; M. J. Sharp, W. Cheng, V. Snieckus in *Tetrahedron Lett.* **1987,** *28*, 5093; G. W. Gray in *J. Chem. Soc. Perkin Trans. II* **1989,** 2041 sowie *Mol. Cryst. Liq. Cryst.* **1989,** *172*, 165, *ibid.* **1991,** *204,* 43 und *ibid.* **1991,** *204,* 91; N. A. Bumagin; E. V. Luzikova, I. P. Letskaya, *Zh. Org. Khim.* **1995,** *31*, 1657-1662; EP-A 0 449 015; WO-A 89/12039; WO-A 89/03821; EP-A 0 354 434 für die direkte Verknüpfung von Aromaten und Heteroaromaten; DE-A 32 01 721 für Verbindungen mit $-CH_2CH_2-$Brückengliedern und K. Seto et al. in *Liq. Cryst.* **1990,** *8*, 861-870 für Verbindungen mit $-C{\equiv}C-$Brückengliedern.

Ester der Formel (I) können auch durch Veresterung der entsprechenden Carbonsäuren (oder ihrer reaktionsfähigen Derivate) mit Alkoholen bzw. Phenolen (oder ihren reaktionsfähigen Derivaten) erhalten werden. Geeignet hierfür ist z. B. auch die sogenannte DCC-Methode (DCC = Dicyclohexylcarbodiimid; vgl.: B. Neises, W. Steglich, *Angew. Chem.* **1978,** *90,* 556-557).

Die entsprechenden Carbonsäuren und Alkohole bzw. Phenole sind entweder bekannt oder können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, z. B. auch gemischte Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. -phenolate, vorzugsweise eines Alkalimetalls wie Natrium oder Kalium, in Betracht.

Die Veresterung wird vorteilhaft in Gegenwart eines inerten Lösungsmittels durchgeführt. Gut geeignet sind insbesondere Ether, wie Diethylether, Di-*n*-butylether, Tetrahydrofuran, Dioxan oder Anisol, Ketone, wie Aceton, Butanon oder Cyclohexanon, Amide, wie Dimethylformamid oder Hexamethylphosphorsäuretriamid, Kohlenwasserstoffe, wie Benzol, Toluol oder Xylol, Halogenkohlenwasserstoffe, wie Tetrachlorkohlenstoff, Dichlormethan oder Tetrachlorethylen und Sulfoxide, wie Dimethylsulfoxid oder Sulfolan.

Ester der Formel (I) lassen sich auch aus den entsprechenden Carbonsäuresalzen (Herstellung analog DE-C 4304756) durch Umsetzung mit Phenolen nach DE-A 4427198 erhalten.

Ether der Formel (I) sind auch durch Veretherung entsprechender Hydroxyverbindungen, vorzugsweise entsprechender Phenole, erhältlich, wobei die Hydroxyverbindung zweckmäßig zunächst in ein entsprechendes Metallderivat, z. B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, Alkylsulfonat oder Dialkylsulfat umgesetzt werden, zweckmäßig in einem inerten Lösungsmittel wie Aceton, 1,2-Dimethoxyethan, Dimethylformamid oder Dimethylsulfoxid, oder auch mit einem Überschuß an wäßriger oder wäßrig-alkoholischer NaOH oder KOH bei Temperaturen zwischen etwa 20°C und 100°C.

Geeignet zur Veretherung ist z. B. auch die Umsetzung von Phenolen mit Alkoholen unter Verwendung von Azodicarbonsäurediethylester und Triphenylphosphin nach der sogenannten *Mitsunobu*-Methode (vgl.: O. Mitsunobu, *Synthesis* **1981,** 1-28 oder L. Navailles, H. T. Nguyen, P. Barois, *Liq. Cryst.* **1996,** *20,* 653-664).

Die entsprechenden Alkohole, Alkylhalogenide, Alkylsulfonate und Dialkylsulfate sind entweder bekannt oder lassen sich in Analogie zu bekannten Verfahren herstellen.

Was die Synthese spezieller Reste $R^1$ und $R^2$ angeht, sei zusätzlich beispielsweise verwiesen auf:

EP-B 0 355 008 für Verbindungen mit siliziumhaltigen Seitenketten,
EP-B 0 292 954 für optisch aktive Verbindungen mit Oxiranestereinheit,
EP-B 0 263 437 für optisch aktive Verbindungen mit Oxiranethereinheit,
US 5,051,506 für optisch aktive Verbindungen mit 2,3-Difluoralkyloxy-Einheit,
US 4,798,680 für optisch aktive Verbindungen mit 2-Fluoralkyloxy-Einheit,
EP-A 0 552 658 für Verbindungen mit Cyclohexylpropionsäureresten.

Die erfindungsgemäßen Verbindungen der Formel (I) eignen sich vorzugsweise für den Einsatz in smektischen und nematischen Flüssigkristallmischungen, im Falle von nematischen Mischungen vorzugsweise für "Active Matrix Displays" (AM-LCD) (siehe z.B. C. Prince, Seminar Lecture Notes, Volume I, p. M-3/3-M-22, SID International Symposium 1997, B.B. Bahadur, Liquid Crystal Applications and Uses, Vol 1, p. 410, World Scientific Publishing, 1990, E. Lüder, Recent Progress of AM LCD's, Proceedings of the $15^{th}$ International Displays Research Conference, 1995, p. 9-12) und "in-plane-switching Displays" (IPS-LCD), im Falle von smektischen Flüssigkristallmischungen vorzugsweise für chirale geneigt smektische (ferroelektrische oder antiferroelektrische) Displays, für ECB-Displays (Electrically Controlled Birefringence) und für elektrokline Displays.

Gegenstand der Erfindung ist auch die Verwendung von Verbindungen der Formel (I) in Flüssigkristallmischungen, vorzugsweise smektischen und nematischen, besonders bevorzugt ferroelektrischen. Insbesondere bevorzugt ist die Verwendung in ferroelektrischen Flüssigkristallmischungen, die im Inverse-Mode betrieben werden.

Weiterhin Gegenstand der Erfindung sind Flüssigkristallmischungen, vorzugsweise smektische und nematische, besonders bevorzugt ferroelektrische und antiferroelektrische, insbesondere ferroelektrische, enthaltend eine oder mehrere Verbindungen der Formel (I).

Die erfindungsgemäßen smektischen oder nematischen Flüssigkristallmischungen eignen sich vorzugsweise für den Einsatz in elektrooptischen Displays, im Falle von nematischen Mischungen besonders für "Active Matrix Displays" und "In-plane-switching Displays" (IPS-LCD), im Falle von smektischen Flüssigkristallmischungen für ECB-Displays (Electrically Controlled Birefringence), für elektrokline Displays und chirale geneigt smektische (ferroelektrische oder antiferroelektrische) Displays.

Die erfindungsgemäßen Flüssigkristallmischungen enthalten im allgemeinen 2 bis 35, vorzugsweise 2 bis 25, besonders bevorzugt 2 bis 20 Komponenten.

Sie enthalten im allgemeinen 0,01 bis 80 Gew.-%, vorzugsweise 0,1 bis 60 Gew.-%, besonders bevorzugt 0,1 bis 30 Gew.-%, an einer oder mehreren, vorzugsweise 1 bis 10, besonders bevorzugt 1 bis 5, ganz besonders bevorzugt 1 bis 3, der erfindungsgemäßen Verbindungen der Formel (I).

Weitere Komponenten von Flüssigkristallmischungen, die erfindungsgemäße Verbindungen der Formel (I) enthalten, werden vorzugsweise ausgewählt aus den bekannten Verbindungen mit smektischen und/oder nematischen und/oder cholesterischen Phasen. Dazu gehören z. B.:

- Derivate des Phenylpyrimidins, wie beispielsweise in WO-A 86/06401 und US-4 874 542 beschrieben,
- metasubstituierte Sechsringaromaten, wie beispielsweise in EP-A 0 578 054 beschrieben,
- Siliziumverbindungen, wie beispielsweise in EP-A 0 355 008 beschrieben,
- mesogene Verbindungen mit nur einer Seitenkette, wie beispielsweise in EP-A 0 541 081 beschrieben,
- Hydrochinonderivate, wie beispielsweise in EP-A 0 603 786 beschrieben,
- Phenylbenzoate, wie beispielsweise bei P. Keller, Ferroelectrics 1984, 58, 3 und J. W. Goodby et al., Liquid Crystals and Ordered Fluids, Bd. 4, New York 1984 beschrieben, und
- Thiadiazole, wie beispielsweise in EP-A 0 309 514 beschrieben.

Als chirale, nicht racemische Dotierstoffe kommen beispielsweise in Frage:

- optisch aktive Phenylbenzoate, wie beispielsweise bei P. Keller, Ferroelectrics 1984, 58, 3 und J. W. Goodby et al., Liquid Crystals and Ordered Fluids, Bd. 4, New York 1984 beschrieben,
- optisch aktive Oxiranether, wie beispielsweise in EP-A 0 263 437 und WO-A 93/13093 beschrieben,
- optisch aktive Oxiranester, wie beispietsweise in EP-A 0 292 954 beschrieben,
- optisch aktive Dioxolanether, wie beispielsweise in EP-A 0 351 746 beschrieben,
- optisch aktive Dioxolanester, wie beispielsweise in EP-A 0 361 272 beschrieben,
- optisch aktive Tetrahydrofuran-2-carbonsäureester, wie beispielsweise in EP-A 0 355 561 beschrieben, und
- optisch aktive 2-Fluoralkylether, wie beispielsweise in EP-A 0 237 007, EP-A 0 428 720 und US-5,051,506 beschrieben.

Geeignete weitere Mischungskomponenten sind insbesondere in der internationalen Patentanmeldung WO-A 97/04039 aufgeführt, auf die hiermit ausdrücklich Bezug genommen wird.

Bevorzugte weitere Komponenten von FLC-Mischungen, die im Inverse-Mode eingesetzt werden, sind:

Phenanthrenderivate der Formel (II),

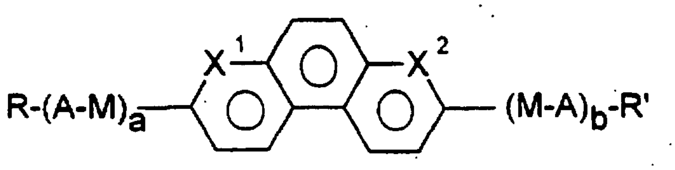

( II )

Fluorpyridine der Formel (III),

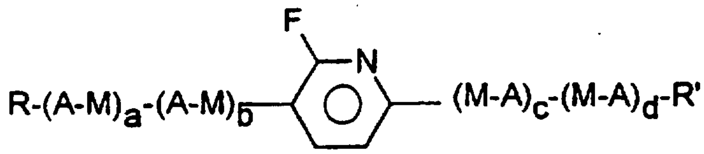

(III)

Difluorphenylenderivate der Formel (IV)

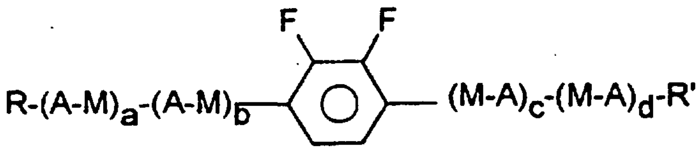

(IV)

Metasubstituierte aromatische Verbindungen der Formel (V)

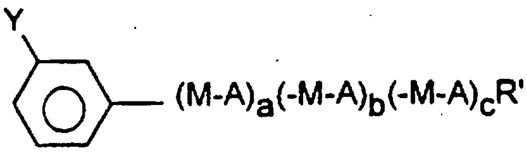

(V)

4-Cyanocyclohexyle der Formel (VI)

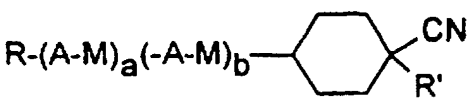

(VI)

1,3,4-Thiadiazole der Formel (VII),

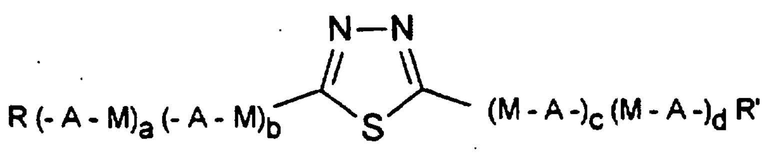

(VII)

wobei die Symbole und Indizes die folgenden Bedeutungen haben:

$X^1$, $X^2$ sind gleich oder verschieden unabhängig voneinander CH, CF oder N;
Y ist F, $CF_3$ oder R;
R, R' haben, gleich oder verschieden, unabhängig voneinander die gleichen Bedeutungen wie $R^1$, $R^2$ in Formel (I);
A, M haben, gleich oder verschieden, unabhängig voneinander die gleichen Bedeutungen wie in Formel (I) und
a,b,c,d sind gleich, oder verschieden, unabhängig voneinander 0 oder 1, mit der Maßgabe, daß die Verbindungen nicht mehr als vier Ringsysteme enthalten dürfen
und, mit Ausnahme der Formel (II), mindestens zwei Ringsysteme enthalten müssen.

Die Mischungen wiederum können Anwendung finden in elektrooptischen oder vollständig optischen Elementen, z. B. Anzeigeelementen, Schaltelementen, Lichtmodulatoren, Elementen zur Bildbearbeitung und/oder Signalverarbeitung oder allgemein im Bereich der nichtlinearen Optik.

Ferner sind die Mischungen für Feldbehandlung, d. h. zum Betrieb in der Quasi-Bookshelf-Geometrie (QBG) (siehe z. B. H. Rieger et al., SID 91 Digest (Anaheim) 1991, 396) geeignet.

Die erfindungsgemäßen ferroelektrischen Flüssigkristallmischungen eignen sich insbesondere zum Betrieb im sogenannten Inverse- oder $TV_{(min)}$-Mode (siehe z.B.: J. C. Jones, M. J. Towler, J. R. Hughes, Displays 1993, 14,

Nr. 2, 86-93; M. Koden, Ferroelectrics 1996, 179, 121-129).

Flüssigkristalline Mischungen, die Verbindungen der allgemeinen Formel (I) enthalten, sind besonders für die Verwendung in elektrooptischen Schalt- und Anzeigevorrichtungen (Displays) geeignet. Diese Displays sind üblicherweise so aufgebaut, daß eine Flüssigkristallschicht beiderseitig von Schichten eingeschlossen ist, die üblicherweise, in dieser Reihenfolge ausgehend von der LC-Schicht, mindestens eine Orientierungsschicht, Elektroden und eine Begrenzungsscheibe (z. B. aus Glas) sind. Darüberhinaus können sie Abstandshalter, Kleberahmen, Polarisatoren sowie für Farbdisplays dünne Farbfilterschichten enthalten. Weitere mögliche Komponenten sind Antireflex-, Passivierungs-, Ausgleichs- und Sperrschichten sowie elektrisch-nichtlineare Elemente, wie Dünnschichttransistoren (TFT) und Metall-Isolator-Metall-(MIM)-Elemente. Im Detail ist der Aufbau von Flüssigkristalldisplays bereits in einschlägigen Monographien beschrieben (siehe z. B. E. Kaneko, "Liquid Crystal TV Displays: Principles and Applications of Liquid Crystal Displays", KTK Scientific Publishers, 1987).

Ein weiterer Gegenstand der Erfindung ist daher eine Schalt- undloder Anzeigevorrichtung, vorzugsweise eine smektische oder nematische, insbesondere eine ferroelektrische, enthaltend eine Flüssigkristallmischung, die eine oder mehrere Verbindungen der Formel (I) enthält.

Bei Vorichtungen, die eine nematische Flüssigkristallmischung enthalten sind "Active Matrix Displays" und "In-plane-switching Displays" (IPS-LCD) bevorzugt.

Bei Vorrichtungen, die eine smektische Flüssigkristallmischung enthalten, sind ECB-Displays (Electrically Controlled Birefringence), elektrokline Displays und chirale geneigt smektische (ferroelektrische oder antiferroelektrische) Displays bevorzugt.

Solche Vorrichtungen können beispielsweise als Computerdisplays oder in Chipkarten Anwendung finden.

Vorzugsweise wird eine erfindungsgemäße ferroelektrische Schalt- undloder Anzeigevorrichtung im Normal- oder Inverse-Mode betrieben.

Durch Multiplexansteuerung betriebene ferroelektrische Schalt- und/oder Anzeigevorrichtungen können unter anderem auf zwei unterschiedliche Weisen betrieben werden, die sogenannte normale (Normal-Mode) oder die sogenannte inverse (Inverse Mode auch $\tau V_{(min)}$-Mode). Der Unterschied zwischen beiden liegt im Ansteuerschema und in den verschiedenen Anforderungen an den dielektrischen Tensor des FLC-Materials, d. h. der FLC-Mischung. Einen Überblick geben z. B. J. C. Jones et al. in Displays 1993, 14, Nr. 2, 86-93 im folgenden als "Jones" bezeichnet, und M. Koden in Ferroelectrics 1996, 179, 121-129, sowie die dort aufgeführte Literatur.

Die Schaltcharakteristika einer FLC-Vorrichtung können im allgemeinen durch ein Diagramm dargestellt werden, bei dem die Treiberspannung (V) horizontal und die Breite der Ansteuerpulse ($\tau$, Zeit) vertikal aufgetragen sind (siehe z. B. Jones, Abb. 4, 8, 10 und 11).

Eine Schaltkurve wird experimentell bestimmt und teilt die V, $\tau$-Fläche in einen schaltenden- und einen nicht schaltenden Bereich. Üblicherweise verkürzt sich bei Erhöhung der Spannung die Pulsbreite. Dieses Verhalten kennzeichnet den sogenannten Normal-Mode (siehe z. B. Jones, Abb. 4).

Bei geeigneten Materialien weist die $V\tau$-Kurve jedoch ein Minimum auf (bei der Spannung $V_{(min)}$), wie z. B. bei Jones in den Abbildungen 8, 10 und 11 zu sehen. Dieses Minimum kommt durch Überlagerung von dielektrischer und ferroelektrischer Verdrillung zustande. FLC-Vorrichtungen werden im Inverse-Mode betrieben, wenn die Summe der Zeilen- und Spalten-Treiberspannung im Arbeitstemperaturbereich höher als das Minimum auf der VT-Kurve sind, d. h. $V_{(Zelle)} + V_{(Spalte)} > V_{(min)}$.

In der vorliegenden Anmeldung sind verschiedene Dokumente zitiert, beispielsweise um das technische Umfeld der Erfindung zu illustrieren.

Auf den Inhalt der deutschen Patentanmeldung 196 53 009.1, deren Priorität die vorliegende Anmeldung beansprucht, wird hiermit ausdrücklich Bezug genommen.

Die Erfindung wird durch die nachfolgenden Beispiele weiter erläutert.

Beispiel 1:

6-Fluor-3-octyl-8-hexyloxyphenanthridin

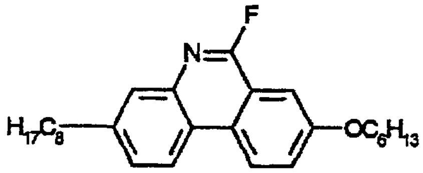

Aus 3-Brom-6-fluor-8-hexyloxyphenanthridin, Octylmagnesiumbromid und 1,3-Bis(diphenylphosphino)propan-nickel(II)-chlorid (dppp-$NiCl_2$) in THF.

Beispiel 2:

6-Fluor-8-(2-(*S*)-fluoroctyloxy)-3-octylphenanthridin

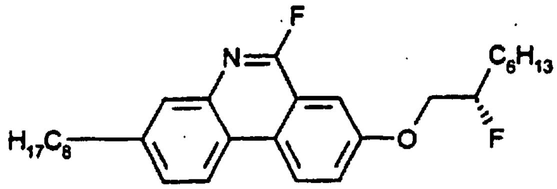

Aus 6-Fluor-3-octylphenanthridin-8-ol durch Umsetzung mit 2-*(S)*-Fluoroctan-1-ol mittels Mitsunobu-Reaktion.

Beispiel 3:

3-[4-(Butyldimethylsilanyl)-butyloxy]-6-fluor-8-hexyloxyphenanthridin

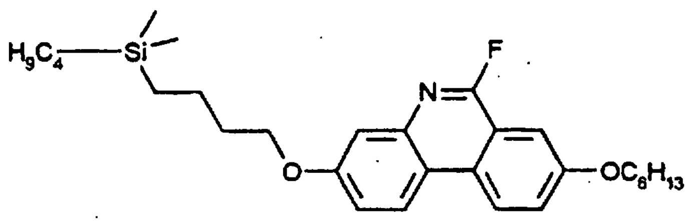

Aus 6-Fluor-8-hexyloxyphenanthridin-3-ol durch Umsetzung mit 4-(Butyldimethylsilanyl)-butan-1-ol mittels Mitsunobu-Reaktion.

Beispiel 4:

*trans*-4-Pentylcyclohexancarbonsäure-(6-fluor-3-octylphenanthridin-8-yl)-ester

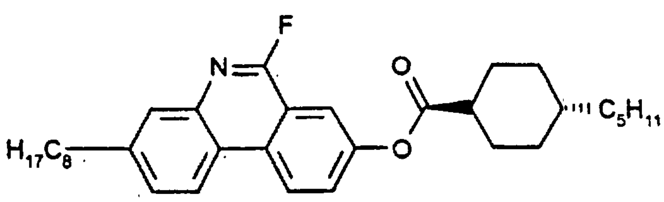

Aus 6-Fluor-3-octylphenanthridin-8-ol durch Umsetzung *trans*-4-Pentylcyclohexancarbonsäurechlorid.

Beispiel 5:

Kohlensäure-(6-fluor-8-octylphenanthridin-3-yl)-ester-ethyl-ester

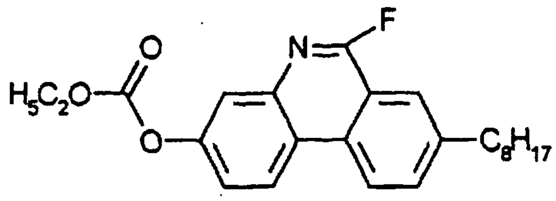

Aus 6-Fluor-8-octylphenanthridin-3-ol durch Umsetzung mit Chlorkohlensäureethylester.

Beispiel 6:

6-Fluor-3-(4-hexyloxyphenyl)-8-octylphenanthridin

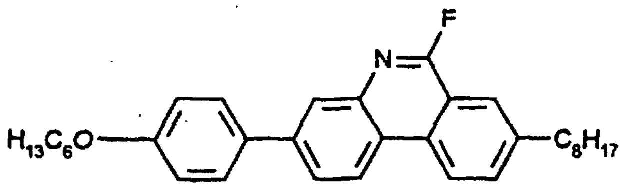

Aus 3-Brom-6-fluor-8-octylphenanthridin durch Umsetzung mit 4-Hexyloxyphenylboronsäure, Natriumcarbonat und Tetrakis(triphenylphosphin)palladium(0) in Toluol/Ethanol/$H_2O$.

Beispiel 7:

8-(4-Decyloxy-3-fluorphenyl)-6-fluor-3-hexyloxyphenanthridin

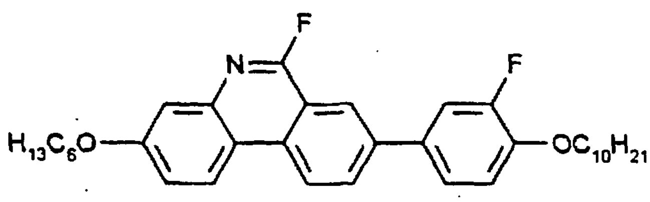

Aus 8-Brom-6-fluor-3-hexyloxyphenanthridin durch Umsetzung mit 4-Decyloxy-3-fluorphenylboronsäure, Natriumcarbonat und

Tetrakis(triphenylphosphin)palladium(0) in Toluol/Ethanol/$H_2O$.

**Patentansprüche**

**1.** 6-Fluorphenanthridin-Derivat der Formel (I),

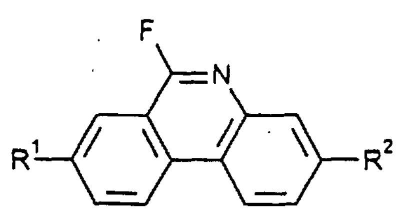

(Ia)

in der die Symbole folgende Bedeutungen haben:
$R^1$,$R^2$ sind gleich oder verschieden
ein geradkettiger oder verzweigter Alkylrest (mit oder ohne asymmetrisches C-Atom) mit 1 bis 16 C-Atomen, wobei

b1) eine oder zwei nicht benachbarte und nicht terminale $CH_2$-Gruppen durch -O-, -CO-O-, -O-CO-, -O-CO-O- oder -$Si(CH_3)_2$-ersetzt sein können und/oder
b2) eine $CH_2$-Gruppe durch 1,4-Phenylen oder trans-1,4-Cyclohexylen ersetzt sein kann und/oder
b3) ein oder mehrere H-Atome durch -F ersetzt sein können und/oder
b4) die terminale $CH_3$-Gruppe durch eine der folgenden chiralen Gruppen (optisch aktiv oder racemisch) ersetzt sein kann:

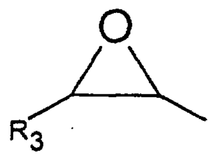
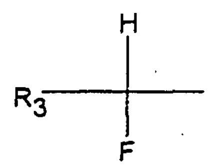
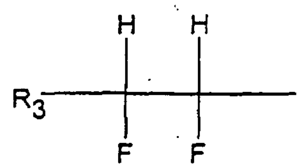

$R^3$ ist

a) Wasserstoff,
b) ein geradkettiger oder verzweigter Alkylrest (mit oder ohne asymmetrisches C-Atom) mit 1-14 C-Atomen, wobei
b1) eine nicht terminale $CH_2$-Gruppe durch -O- ersetzt sein kann.

**2.** Verwendung von 6-Fluorphenanthridin-Derivaten gemäß Anspruch 1 als Komponenten flüssigkristalliner Mischungen.

**3.** Flüssigkristallmischung, enthaltend ein oder mehrere 6-Fluorphenanthridin-Derivate gemäß Anspruch 1.

**4.** Flüssigkristallmischung gemäß Anspruch 3, **dadurch gekennzeichnet, daß** sie ferroelektrisch ist.

**5.** Flüssigkristallmischung gemäß Anspruch 3 oder 4, **dadurch gekennzeichnet, daß** sie 0,01 bis 80 Gew.-% an einem oder mehreren 6-Fluorphenanthridin-Derivaten der Formel (I) enthält.

**6.** Ferroelektrische Schalt- und/oder Anzeigevorrichtung, enthaltend eine ferroelektrische Flüssigkristallmischung gemäß Anspruch 4 oder 5.

**7.** Ferroelektrische Schalt- und/oder Anzeigevorrichtung gemäß Anspruch 6, **dadurch gekennzeichnet, daß** sie im $\tau V_{(min)}$-Mode betrieben wird.

## Claims

**1.** A 6-fluorophenanthridine derivative of the formula (I)

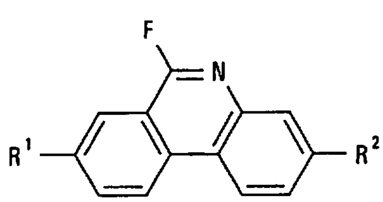 (I)

in which the symbols have the following meanings:

$R^1$ and $R^2$ are identical or different and are a straight-chain or branched alkyl radical (with or without an asymmetric carbon atom) having 1 to 16 carbon atoms, where

b1) one or two nonadjacent and nonterminal $CH_2$ groups can be replaced by -O-, -CO-O-, -O-CO-, -O-CO-O- or $-Si(CH_3)_2-$, and/or
b2) one $CH_2$ group can be replaced by 1,4-phenylene or trans-1,4-cyclohexylene, and/or
b3) one or more H atoms can be replaced by -F, and/or
b4) the terminal $CH_3$ group can be replaced by one of the following chiral groups (optically active or racemic):

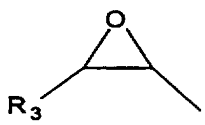

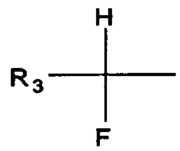

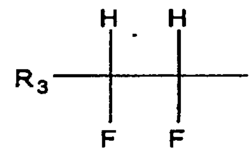

$R^3$ is

a) hydrogen,
b) a straight-chain or branched alkyl radical (with or without an asymmetric carbon atom) having 1-14 carbon atoms, where
b1) one nonterminal $CH_2$ group can be replaced by -O-.

**2.** The use of a 6-fluorophenanthridine derivative as claimed in claim 1 as a component of liquid-crystalline mixtures.

**3.** A liquid-crystal mixture comprising one or more 6-fluorophenanthridine derivatives as claimed in claim 1.

**4.** A liquid-crystal mixture as claimed in claim 3, which is ferroelectric.

**5.** A liquid-crystal mixture as claimed in claim 3 or 4, which contains from 0.01 to 80% by weight of one or more 6-fluorophenanthridine derivatives of the formula (I).

**6.** A ferroelectric switching and/or display device comprising a ferroelectric liquid-crystal mixture as claimed in claim 4 or 5.

**7.** A ferroelectric switching and/or display device as claimed in claim 6, which is operated in the $\tau V_{(min)}$ mode.

**Revendications**

**1.** Dérivé de 6-fluorophénanthridine de formule (I),

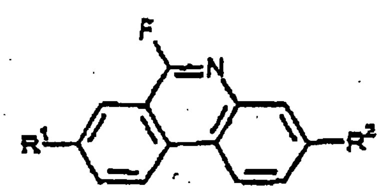

(I)

dans laquelle les symboles ont les significations suivantes :

$R^1$, $R^2$ sont, identiques ou différents, un groupe alkyle à chaîne linéaire ou ramifiée (avec ou sans atome. de

carbone asymétrique). ayant de 1 à 16 atomes de carbone, dans lesquels

b1) un ou deux groupes $CH_2$ non voisins et non terminaux peuvent être remplacés par -O-, -CO-O-, -O-CO-, -O-CO-O- ou $-Si(CH_3)_2-$ et/ou
b2) un groupe $CH_2$ peut être remplacé par un groupe 1,4-phénylène ou trans-1,4-cyclohexylène et/ou
b3) un ou plusieurs atomes H peuvent être remplacés par F et/ou
b4) le groupe $CH_3$ terminal peut être remplacé par un des groupes chiraux suivants (optiquement actif ou racémique) :

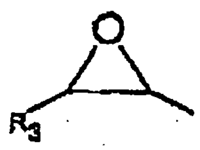
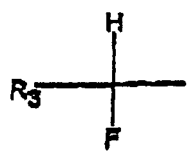
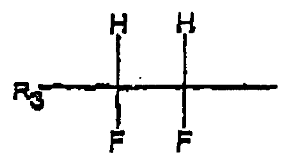

$R^3$ est

a) un atome d'hydrogène ;
b) un groupe alkyle à chaîne linéaire ou ramifiée (avec ou sans atome de carbone asymétrique) ayant. de 1 à 14 atomes de carbone, dans lequel b1) un groupe $CH_2$ non terminal peut être remplacé par -O-.

**2.** Utilisation de dérivés de 6-fluorophénanthridine selon la revendication 1 comme composants de mélanges liquides cristallins.

**3.** Mélange liquide cristallin, contenant un ou plusieurs dérivés de 6-fluorophénanthridine selon la revendication 1.

**4.** Mélange liquide cristallin selon la revendication 3, **caractérisé en ce qu'**il est ferroélectrique.

**5.** Mélange liquide cristallin selon la revendication 3 ou 4, **caractérisé en ce qu'**il contient de 0,01 à 80 % en poids d'un ou de plusieurs dérivés de 6-fluorophénanthridine de formule (I).

**6.** Dispositif de commutation et/ou d'affichage ferroélectrique, contenant un mélange cristallin liquide ferroélectrique selon la revendication 4 ou 5.

**7.** Dispositif de commutation et/ou d'affichage ferroélectrique selon la revendication 6, **caractérisé en ce qu'**il est actionné en mode $\tau V_{(min)}$.